# EUROPEAN PATENT APPLICATION

(11) **EP 2 559 400 A1**
(43) Date of publication of application: **20.02.2013**
(21) Application number: 11006833.5
(22) Date of filing: 19.08.2011
(51) Int. Cl.: A61F 2/06, A61F 2/90, A61F 2/00

(54) **Stent graft with alternating rings**

(71) Applicant: Abbott Laboratories Vascular Enterprises Limited, Dublin 2 (IE)
(72) Inventor: Bregulla, Rainer, 72336 Balingen (DE)
(74) Representative: Peters, Hajo

(57) **Abstract**

The invention relates to a stent graft, adapted for being inserted into a blood vessel, and to a method for forming a stent graft. The stent graft (1) comprises a first stent (2) comprising a plurality of first rings (4) being connected by at least one first connector, wherein the first stent (2) is arranged at least partly in an inner part of the stent graft (1), a second stent (3) comprising a plurality of second rings (5) being connected by at least one second connector, wherein the second stent (3) is arranged at least partly in an outer part of the stent graft (1), and a graft layer is arranged at least partly between the first stent (2) and the second stent (3), wherein the first stent (2) and the second stent (3) having each a predetermined length and the plurality of first rings (4) and the plurality of second rings (5) are axially arranged along the length such that the plurality of second rings (5) fit into the plurality of first rings (4). In this way, a possibility is provided to avoid the problem of integrity in connection with having as less as possible interaction between the first stent and the second stent and thus to minimize the risks of shear effects.

## Description

### Field of the invention

The invention relates to a stent graft, preferably adapted for being inserted into a blood vessel. The invention also relates to a method for forming a stent graft.

### Background of the invention

Document US 6,302,907 B1 describes a generally tubular intraluminal compound stent comprising a plurality of component stents, each component stent having a length and a plurality of individuals hoops axially disposed along the length and connected by connecting spine, wherein the component stents are meshed with one another such that at least one hoop of one component stent is positioned between axially adjacent hoops of another component stent.

A stent is an elongated device used to support an intraluminal wall. In case of a stenosis, a stent provides an unobstructed conduit for blood in the area of the stenosis. An intraluminal prosthesis may comprise a stent that carries a prosthetic graft layer of fabric. Such a prosthesis is used, for instance, to treat a vascular aneurysm by removing the pressure on a weakened part of an artery so as to reduce the risk of rupture. Typically, an intraluminal stent or prosthesis is implanted in a blood vessel at the site of a stenosis or aneurysm endoluminally, i.e. by so-called "minimally invasive techniques" in which the stent, restrained in a radially compressed configuration by a sheath or catheter, is delivered by a stent deployment system to the site where it is required.

A stent graft is a product which can save life during interventional procedures in cases where the blood vessel ruptures or perforates. Typically, a stent is associated with a polytetrafluoroethylene, PTFE for short, or with an expanded PTFE, ePTFE for short.

However, it is a problem of integrity comprising thin places, smaller or larger holes after expansion, which has been observed on the ePTFE in combination with a stent design and the crimp process.

### Summary of the invention

It is the object of the invention to provide a possibility to minimize or even solve the problem of integrity in connection with minimizing the occurrence of defects, such as holes, and the risks of shear effects and/or thin places.

This object is achieved by the subject matter of the independent claims. Preferred embodiments are defined in the sub claims.

According to a first aspect of the invention, this object is achieved by a stent graft, adapted for being inserted into a blood vessel, comprising a first stent comprising a plurality of first rings being connected by at least one first connector, wherein the first stent is arranged at least partly in an inner part of the stent graft, a second stent comprising a plurality of second rings being connected by at least one second connector, wherein the second stent is arranged at least partly in an outer part of the stent graft, and a graft layer is arranged at least partly between the first stent and the second stent, wherein the first stent and the second stent having each a predetermined length and the plurality of first rings and the plurality of second rings are axially arranged along the length such that the plurality of second rings fit into the plurality of first rings.

The term "fitting of the plurality of second rings into the plurality of first rings" refers to the gaps arranged between the plurality of first rings along the length of the first stent and/or to the gaps arranged between the plurality of second rings along the length of the second stent, respectively, which are large to a certain extent such that they match exactly into each other. This preferably minimizes the overlapping and interaction area in the stent graft which results in less or almost no ePTFE defects during a crimping process and/or an expansion process. Preferably, the predetermined length of the first stent and the second stent are equal. However, it is also possible that their lengths differ from each other.

According to a preferred embodiment of the invention, the at least one first connector of the first stent is adapted for connecting the plurality of first rings to one another in a longitudinal direction of the stent graft and the at least one second connector of the second stent is adapted for connecting the plurality of second rings to one another in a longitudinal direction of the stent graft such that the plurality of second rings fits into the plurality of first rings.

Further, according to a preferred embodiment of the invention, the at least one first connector of the first stent comprises a predetermined length which is larger than the distance between a peak and a valley of a second ring of the second stent, and wherein the at least one second connector of the second stent comprises a predetermined length which is larger than the distance between a peak and a valley of a first ring of the first stent. The predetermined length of the at least one first connector is preferably larger than the distance between a peak and a valley of a second ring of the second stent by up to a first predetermined factor, and the predetermined length of the at least one second connector is preferably larger than the distance between a peak and a valley of a first ring of the first stent by up to a second predetermined factor, the second predetermined factor being different from the first predetermined factor, both being preferably in a range between 1.1 and 3, more preferably between 1.2 and 2.8, most preferably between 1.25 and 2.5.

Moreover, according to a preferred embodiment of the invention, the first stent and the second stent are meshed with each other such that the plurality of first rings alternates with the plurality of second rings in a longitudinal direction of the stent graft. In this way, the whole construction, i.e. the stent graft, becomes very stable. Preferably, the first stent and the second stent are coaxially arranged to each other.

According to a preferred embodiment of the invention, the first stent and the second stent are radially compressible and/or expandable. Therefore, their structures can easily adapt themselves to be in compliance with the surrounding conditions together with the respective requirements. The overlapping and interaction area of the stent graft is preferably arranged at least partly between the plurality of first rings and the plurality of second rings such that degradation of the graft layer is minimized during a crimping and/or an expansion process. The graft layer preferably minimizes the interaction between the first stent and the second stent. The material of the graft layer is preferably radially expandable up to a predetermined factor of its initial size. The material of the graft layer preferably comprises ePTFE and/or a fluoropolymer.

According to a preferred embodiment of the invention, the graft layer comprises a tubular shape and is arranged between the first stent and the second stent, each stent comprises a tubular shape. The material of this first stent and/or the second stent preferably comprises stainless steel. Stainless steel preferably comprises cobalt chromium. Preferably, the material of the first stent and/or the second stent is flexible.

According to a second aspect of the invention, above mentioned object is achieved by a method for forming a stent graft comprising the steps: forming a first stent with a plurality of first rings being connected by at least one first connector and positioning the first stent at least partly in an inner part of the stent graft, forming a second stent with a plurality of second rings being connected by at least one second connector and positioning the second stent at least partly in an outer part of the stent graft, and forming a graft layer and positioning the graft layer at least partly between the first stent and the second stent, wherein the first stent and the second stent having each a predetermined length and the plurality of first rings and the plurality of second rings are axially arranged along the length such that the plurality of second rings fit into the plurality of first rings.

It is an idea of the invention to provide a stent graft, which preferably comprises a sandwich construction. Preferably, the expandable PTFE graft material is placed at least partly between two flexible stainless steel stents. This preferably provides seamless attachment of the graft material. It is worth noting that the PTFE graft material is adapted for being integrated into a stent graft system. The stent graft preferably seals off the vessel wall, for instance in perforations and ruptures, but also in aneurysms, dissections, and fistulas. Preferably, the stent graft is crimped down to a compatibility of 7F and of 8F introducer sheaths. This is preferably chosen according to the diameter and profile of the chosen catheter. The stent graft preferably shows predictable foreshortening when expanded. This, together with high radiopacity, facilitates exact placement of the stent. Advantageously, the stent graft shows excellent radial strength when expanded. The material used, such as cobalt chromium, and/or the stent geometry applied preferably allow for a flexible stent platform without comprising strength.

### Brief description of the drawings

These and other aspects of the invention will be apparent from and elucidated with reference to the embodiments described hereinafter.

In the drawings:
- Fig. 1: illustrates a stent graft assembly according to a first preferred embodiment of the invention;
- Fig. 2: shows a stent graft design according to the first preferred embodiment of the invention;
- Fig. 3: shows an expanded peripheral stent graft with holes;
- Fig. 4: shows a superposition of an inner stent and an outer stent design according to a second preferred embodiment of the invention;
- Fig. 5: shows a stent design in expanded condition with alternating inside and outside rings according to a third preferred embodiment of the invention; and
- Fig. 6: shows a stent design in expanded configuration with minor imprint of the stent into the ePTFE according to a fourth preferred embodiment of the invention.

### Detailed description of embodiments

The stent graft according to the invention is preferably produced using a sandwich construction. The expandable PTFE graft material is preferably arranged between two flexible stainless steel stents according to a first preferred embodiment of the invention. The stent design is preferably made of cobalt chromium material, CoCr material for short.

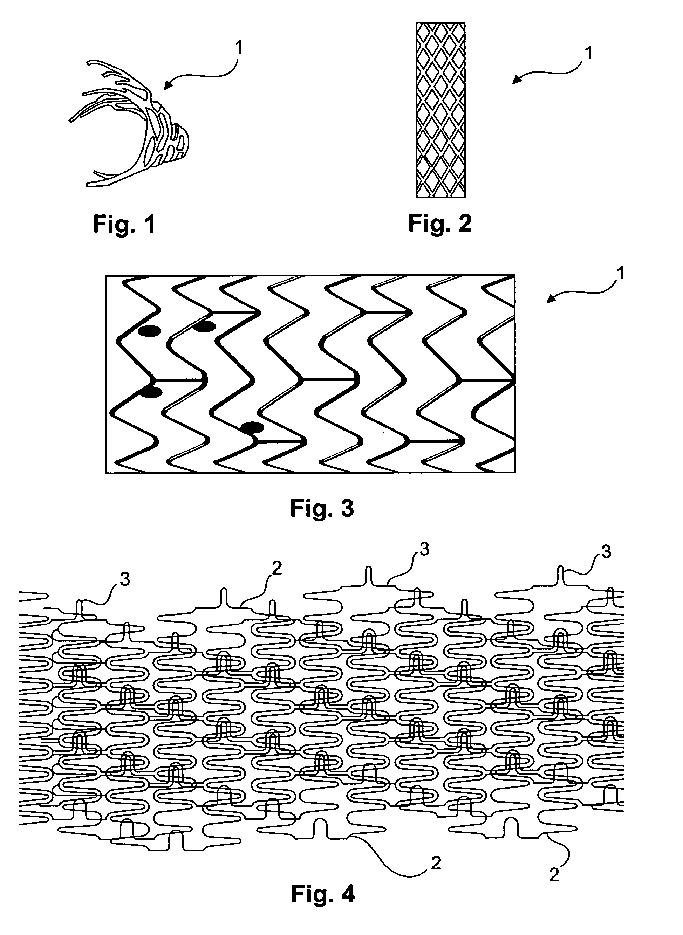

Figs. 1 and 2 illustrate a stent graft assembly 1 and a stent graft design 1, respectively, according to a first preferred embodiment of the invention. The design input specification is to be 7F compatible with a large stent design which shows typically a diameter of 10 mm and requires a smaller crimp profile. Preferably, this profile is achieved with a high crimp pressure and the ePTFE layer will be more compressed between the two stents which may lead to thin places. According to the first preferred embodiment of the invention, these thin spots and the additional intersection areas of the inner and outer stent in the stent graft 1, corresponding to the first and second stent, respectively, during the expansion will lead into defects, such as holes. This is due to the shear effect which the person skilled in the art knows.

Fig. 3 shows a stent graft 1 in which thin spots and the additional intersection areas of the inner and outer stent during the expansion lead into the defects, such as holes, due to the shear effect.

It is an idea of the invention to have as less as possible interaction between the inner stent and the outer stent, i.e. between the first stent, preferably arranged at least partly in an inner part of the stent graft 1, and the second stent, preferably arranged at least partly in an outer part of the stent graft. Thus, the risks of shear effects or thin places on the ePTFE are minimized.

According to a second preferred embodiment of the invention, the stent graft 1 comprises a first stent 2 and a second stent 3, each comprising a plurality of rings. The first stent 2 corresponds to the inner stent and the second stent 3 corresponds to the outer stent, respectively, according to this preferred embodiment of the invention. The gaps of the rings of the outer or inner stent are large such that they fit exactly into each other, as is illustrated in Fig. 4.

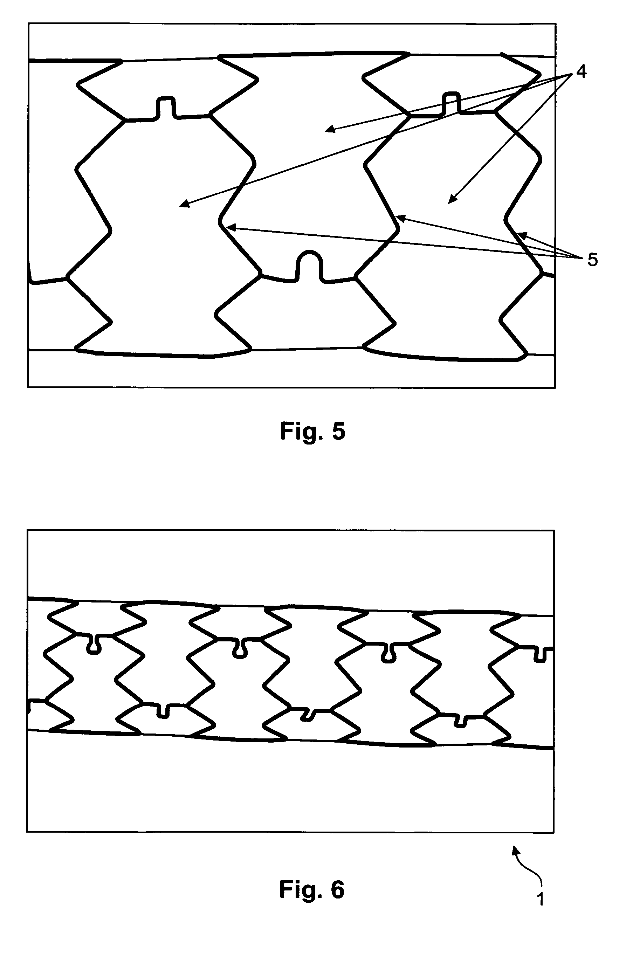

According to a third and fourth preferred embodiment of the invention, the overlapping and interaction area is minimized due to a graft layer which is arranged at least partly between the first stent and the second stent. This results in less or no ePTFE defects during a crimping and/ or an expansion process. The stent graft in expanded condition with alternating inside and outside rings is illustrated in Fig. 5. The first stent comprises a plurality of first rings 4 and the second stent comprises a plurality of second rings 5, which fit exactly into each other in this third preferred embodiment of the invention. Fig. 6 shows a stent graft 1 in expanded configuration with a minor imprint of the stent into the ePTFE in this fourth preferred embodiment of the invention.

While the invention has been illustrated and described in detail in the drawings and foregoing description, such illustration and description are to be considered illustrative or exemplary and not restrictive and it is not intended to limit the invention to the disclosed embodiments. The mere fact that certain measures are recited in mutually different dependent claims does not indicate that a combination of these measures cannot be used advantageously.

## Claims

1. A stent graft (1), adapted for being inserted into a blood vessel, comprising:
a first stent (2) comprising a plurality of first rings (4) being connected by at least one first connector, wherein the first stent (2) is arranged at least partly in an inner part of the stent graft (1),
a second stent (3) comprising a plurality of second rings (5) being connected by at least one second connector, wherein the second stent (3) is arranged at least partly in an outer part of the stent graft (1), and
a graft layer is arranged at least partly between the first stent (2) and the second stent (3),
wherein the first stent (2) and the second stent (3) having each a predetermined length and the plurality of first rings (4) and the plurality of second rings (5) are axially arranged along the length such that the plurality of second rings (5) fit into the plurality of first rings (4).

2. The stent graft (1) according to claim 1, wherein the at least one first connector of the first stent (2) is adapted for connecting the plurality of first rings (4) to one another in a longitudinal direction of the stent graft (1) and the at least one second connector of the second stent (3) is adapted for connecting the plurality of second rings (5) to one another in a longitudinal direction of the stent graft (1) such that the plurality of second rings (5) fits into the plurality of first rings (4).

3. The stent graft (1) according to claim 2, wherein the at least one first connector of the first stent (2) comprises a predetermined length which is larger than the distance between a peak and a valley of a second ring (5) of the second stent (3), and wherein the at least one second connector of the second stent (3) comprises a predetermined length which is larger than the distance between a peak and a valley of a first ring (4) of the first stent (2).

4. The stent graft (1) according to any of the preceding claims, wherein the first stent (2) and the second stent (3) are meshed with each other such that the plurality of first rings (4) alternates with the plurality of second rings (5) in a longitudinal direction of the stent graft (1).

5. The stent graft (1) according to any of the preceding claims, wherein the first stent (2) and the second stent (3) are coaxially arranged to each other.

6. The stent graft (1) according to any of the preceding claims, wherein the first stent (2) and the second stent (3) are radially compressible and/or expandable.

7. The stent graft (1) according to any of the preceding claims, wherein the overlapping and interaction area of the stent graft (1) is arranged at least partly between the plurality of first rings (4) and the plurality of second rings (5) such that degradation of the graft layer is minimized during a crimping and/or an expansion process.

8. The stent graft (1) according to any of the preceding claims, wherein the material of the graft layer is radially expandable up to a predetermined factor of its initial size.

9. The stent graft (1) according to any of the preceding claims, wherein the material of the graft layer comprises ePTFE and/or a fluoropolymer.

10. The stent graft (1) according to any of the preceding claims, wherein the graft layer comprises a tubular shape and is arranged between the first stent (2) and the second stent (3), each stent (2, 3) comprises a tubular shape.

11. The stent graft (1) according to any of the preceding claims, wherein the material of the first stent (2) and/or the second stent (3) comprises stainless steel.

12. The stent graft (1) according to claim 11, wherein stainless steel comprises cobalt chromium.

13. The stent graft (1) according to any of the preceding claims, wherein the material of the first stent (2) and/or the second stent (3) is flexible.

14. A method for forming a stent graft (1) comprising the steps:
forming a first stent (2) with a plurality of first rings (4) being connected by at least one first connector and positioning the first stent (2) at least partly in an inner part of the stent graft (1),
forming a second stent (3) with a plurality of second rings (5) being connected by at least one second connector and positioning the second stent (3) at least partly in an outer part of the stent graft (1), and
forming a graft layer and positioning the graft layer at least partly between the first stent (2) and the second stent (3),
wherein the first stent (2) and the second stent (3) having each a predetermined length and the plurality of first rings (4) and the plurality of second rings (5) are axially arranged along the length such that the plurality of second rings (5) fit into the plurality of first rings (4).
